# EUROPEAN PATENT SPECIFICATION

(11) **EP 1 987 029 B1**
(45) Date of publication and mention of the grant of the patent: **13.01.2010**
(21) Application number: 07704570.6
(22) Date of filing: 13.02.2007
(51) Int. Cl.: C07D 453/02, A61K 31/501, A61P 25/00

(54) **ENANTIOPURE QUINUCLIDINYLOXY PYRIDAZINES AND THEIR USE AS NICOTINIC ACETYLCHOLINE RECEPTOR LIGANDS**
ENANTIOMERENREINE CHINUCLIDINYLOXY-PYRIDAZINE UND IHRE VERWENDUNG ALS NIKOTIN-ACETYLCHOLIN-REZEPTORLIGANDEN
QUINUCLIDINYLOXY PYRIDAZINES ÉNANTIOPURES ET LEURS UTILISATIONS EN TANT QUE LIGANDS DU RÉCEPTEUR NICOTINIQUE DE L'ACÉTYLCHOLINE

(30) Priority: 16.02.2006 DK 200600224; 17.02.2006 US 774220 P
(43) Date of publication of application: 05.11.2008
(73) Proprietor: NeuroSearch A/S, 2750 Ballerup (DK)
(72) Inventor: PETERS, Dan, DK-2750 Ballerup (DK); TIMMERMANN, Daniel, B., DK-2750 Ballerup (DK); OLSEN, Gunnar, M., DK-2750 Ballerup (DK); NIELSEN, Elsebet, Østergaard, DK-2750 Ballerup (DK); DYHRING, Tino, DK-2750 Ballerup (DK)
(74) Representative: Abildgren, Michael Padkjaer
(86) International application number: PCT/EP2007/051399
(87) International publication number: WO 2007/093602

(56) References cited:
- WO-A-2004/022556
- WO-A-2006/040352
- US-A1- 2005 137 226

## Description

### TECHNICAL FIELD

This invention relates to novel enantiopure azabicyclo pyridazinyloxy derivatives, which are found to be cholinergic ligands at the nicotinic acetylcholine receptors and modulators of the monoamine receptors and transporters. Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

### BACKGROUND ART

The endogenous cholinergic neurotransmitter, acetylcholine, exert its biological effect via two types of cholinergic receptors, the muscarinic Acetyl Choline Receptors (mAChR) and the nicotinic Acetyl Choline Receptors (nAChR).

As it is well established that muscarinic acetylcholine receptors dominate quantitatively over nicotinic acetylcholine receptors in the brain area important to memory and cognition, and much research aimed at the development of agents for the treatment of memory related disorders have focused on the synthesis of muscarinic acetylcholine receptor modulators.

Recently, however, an interest in the development of nAChR modulators has emerged. Such nAChR modulators are disclosed in WO 2004/022556 and US 2005/137226. Several diseases are associated with degeneration of the cholinergic system i.e. senile dementia of the Alzheimer type, vascular dementia and cognitive impairment due to the organic brain damage disease related directly to alcoholism. Indeed several CNS disorders can be attributed to a cholinergic deficiency, a dopaminergic deficiency, an adrenergic deficiency or a serotonergic deficiency.

### SUMMARY OF THE INVENTION

The present invention is devoted to the provision novel modulators of the nicotinic and/or of the monoamine receptors, which modulators are useful for the treatment of diseases or disorders related to the cholinergic receptors, and in particular the nicotinic acetylcholine receptor (nAChR), the serotonin receptor (5-HTR), the dopamine receptor (DAR) and the norepinephrine receptor (NER), and of the biogenic amine transporters for serotonin (5-HT), dopamine (DA) and norepinephrine (NE).

Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

The compounds of the invention may also be useful as diagnostic tools or monitoring agents in various diagnostic methods, and in particular for *in vivo* receptor imaging (neuroimaging), and they may be used in labelled or unlabelled form.

In its first aspect the invention provides novel enantiopure azabicyclo pyridazinyloxy derivatives of Formula I 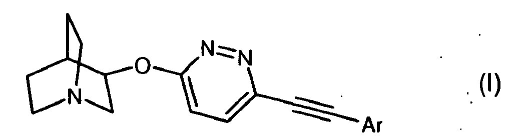 or a pharmaceutically acceptable salt thereof, wherein
Ar represents a phenyl or a thienyl group; which phenyl and thienyl groups are optionally substituted with halo, alkoxy, amino or alkyl-carbonyl-amino.

In its second aspect the invention provides pharmaceutical compositions comprising a therapeutically effective amount of the enantiopure azabicyclo pyridazinyloxy derivative of the invention, or a pharmaceutically-acceptable addition salt thereof, or a prodrug thereof, together with at least one pharmaceutically-acceptable carrier or diluent.

In a further aspect the invention relates to the use of the enantiopure azabicyclo pyridazinyloxy derivative of the invention, or a pharmaceutically-acceptable addition salt thereof, for the manufacture of a pharmaceutical composition/medicament for the treatment, prevention or alleviation of a disease or a disorder or a condition of a mammal, including a human, which disease, disorder or condition is responsive to modulation of cholinergic receptors and/or monoamine receptors.

In a final aspect the invention provides methods of treatment, prevention or alleviation of diseases, disorders or conditions of a living animal body, including a human, which disorder, disease or condition is responsive to modulation of cholinergic receptors and/or monoamine receptors, which method comprises the step of administering to such a living animal body in need thereof a therapeutically effective amount of the enantiopure azabicyclo pyridazinyloxy derivative of the invention.

Other objects of the invention will be apparent to the person skilled in the art from the following detailed description and examples.

### DETAILED DISCLOSURE OF THE INVENTION

### Enantiopure Azabicyclo Pyridazinyloxy Derivatives

In a first aspect novel enantiopure azabicyclo pyridazinyloxy derivatives are provided. The enantiopure azabicyclo pyridazinyloxy derivatives of the invention may be represented by the general Formula I 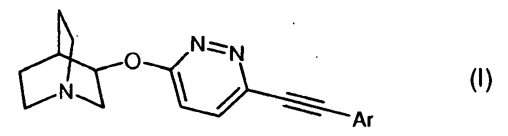 or a pharmaceutically acceptable salt thereof, wherein
Ar represents a phenyl or a thienyl group; which phenyl and thienyl groups are optionally substituted with halo, alkoxy, amino or alkyl-carbonyl-amino.

In a more preferred embodiment the enantiopure azabicyclic pyridazinyloxy derivative of the invention is an enantiopure azabicyclic aryl derivative of Formula la 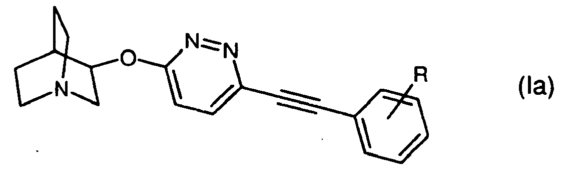 or a pharmaceutically acceptable salt thereof, wherein R represents hydrogen, halo, alkoxy, amino or alkyl-carbonyl-amino.

In an even more preferred embodiment R represents hydrogen, alkoxy, amino or alkyl-carbonyl-amino.

In a still more preferred embodiment R represents hydrogen, fluoro, chloro, bromo, iodo, methoxy, amino or methyl-carbonyl-amino.

In a yet more preferred embodiment R represents hydrogen, methoxy, amino or methyl-carbonyl-amino.

In a most preferred embodiment R represents hydrogen.

In another preferred embodiment the enantiopure azabicyclic pyridazinyloxy derivative of the invention is a compound of Formula I, wherein Ar represents a phenyl group; which phenyl is optionally substituted with halo, alkoxy, amino or alkyl-carbonyl-amino.

In a more preferred embodiment the phenyl group is optionally substituted with alkoxy, amino or alkyl-carbonyl-amino.

In an even more preferred embodiment the phenyl group is optionally substituted with alkoxy, amino or alkyl-carbonyl-amino.

In a most preferred embodiment the phenyl group is optionally substituted with methoxy, amino or methyl-carbonyl-amino.

In a most preferred embodiment Ar represents a phenyl group.

In a third preferred embodiment the enantiopure azabicyclic pyridazinyloxy derivative of the invention is a compound of Formula I, wherein Ar represents a thienyl group; which thienyl is optionally substituted with halo, alkoxy, amino or alkyl-carbonyl-amino.

In a more preferred embodiment the thienyl group is optionally substituted with alkoxy, amino or alkyl-carbonyl-amino.

In an even more preferred embodiment the thienyl group is optionally substituted with methoxy, amino or methyl-carbonyl-amino.

In a most preferred embodiment Ar represents a thienyl group.

In a fourth preferred embodiment the enantiopure azabicyclic pyridazinyloxy derivative of the invention is
(R)-3-(6-Phenylethynyl-pyridazin-3-yloxy)-1-aza-bicyclo[2.2.2]octane;
(R)-3-(6-Thiophen-3-ylethynyl-pyridazin-3-yloxy)-1-aza-bicyclo[2.2.2]octane;
(R)-3-[6-(4-Methoxy-phenylethynyl)-pyridazin-3-yloxy]-1-aza-bicyclo[2.2.2]-octane;
(R)-*N*-{4-[6-(1-Aza-bicyclo[2.2.2]oct-3-yloxy)-pyridazin-3-ylethynyl]-phenyl}-acetamide;
(R)-4-[6-(1-Aza-bicyclo[2.2.2]oct-3-yloxy)-pyridazin-3-ylethynyl]-phenyl-amine;
(R)-3-[6-(1-Aza-bicyclo[2.2.2]oct-3-yloxy)-pyridazin-3-ylethynyl]-phenyl-amine;
(S)-3-(6-Phenylethynyl-pyridazin-3-yloxy)-1-aza-bicyclo[2.2.2]octane;
(S)-3-(6-Thiophen-3-ytethynyl-pyridazin-3-yloxy)-1-aza-bicyclo[2.2.2]octane;
(S)-3-[6-(4-Methoxy-phenylethynyl)-pyridazin-3-yloxy]-1-aza-bicyclo[2.2.2]-octane;
(S)-*N*-{4-[6-(1-Aza-bicyclo[2.2.2]oct-3-yloxy)-pyridazin-3-ylethynyl]-phenyl}-acetamide;
(S)-4-[6-(1-Aza-bicyclo[2.2.2]oct-3-yloxy)-pyridazin-3-ylethynyl]-phenyl-amine; or
(S)-3-[6-(1-Aza-bicyclo[2.2.2]oct-3-yloxy)-pyridazin-3-ylethynyl]-phenyl-amine;
or a pharmaceutically acceptable salt thereof.

In a fifth preferred embodiment the enantiopure azabicyclic pyridazinyloxy derivative of the invention is
(R)-3-(6-Phenylethynyl-pyridazin-3-yloxy)-1-aza-bicyclo[2.2.2]octane;
or a Pharmaceutical acceptable salt thereof.

In a sixth preferred embodiment the enantiopure azabicyclic pyridazinyloxy derivative of the invention is
(S)-3-(6-Phenylethynyl-pyridazin-3-yloxy)-1-aza-bicyclo[2.2.2]octane;
or a pharmaceutically acceptable salt thereof.

Any combination of two or more of the embodiments as described above is considered within the scope of the present invention.

### Definition of Substituents

In the context of this invention halo represents a fluorine, a chlorine, a bromine or an iodine atom.

In the context of this invention an alkoxy group designates an "alkyl-O-" group, wherein alkyl is defined as a univalent saturated, straight of branched hydrocarbon chain, preferably containing of from one seven carbon atoms (C₁₋₇-alkoxy). Examples of preferred alkoxy groups of the invention include methoxy and ethoxy.

In the context of this invention an alkyl-carbonyl-amino group designates an "alkyl-CO-NH-" group, wherein alkyl is defined as a univalent saturated, straight or branched hydrocarbon chain, preferably containing of from one seven carbon atoms (C₁₋₇-alkyl). Preferred alkyl-carbonyl-amino groups of the invention include acetamido.

### Enantiopurity

In the context of this invention a compound being enantiopure means that the compound is in enantiomeric excess of at least 95.0% (w/w) over the opposite enantiomer. In one embodiment, the enantiopure compound is in enantiomeric excess of at least 97.0%, 98.0% or 99.0% over the opposite enantiomer. In a further embodiment, the enantiopure compound is in enantiomeric excess of at least 99.2%, 99.5%, or 99.7% over the opposite enantiomer. In a still further embodiment, the enantiopure compound is in enantiomeric excess of at least 99.90% or 99.95% over the opposite enantiomer.

### Pharmaceutically Acceptable Salts

The azabicyclo pyridazinyloxy derivative of the invention may be provided in any form suitable for the intended administration. Suitable forms include pharmaceutically (i.e. physiologically) acceptable salts, and pre- or prodrug forms of the chemical compound of the invention.

Examples of pharmaceutically acceptable addition salts include, without limitation, the non-toxic inorganic and organic acid addition salts such as the hydrochloride derived from hydrochloric acid, the hydrobromide derived from hydrobromic acid, the nitrate derived from nitric acid, the perchlorate derived from perchloric acid, the phosphate derived from phosphoric acid, the sulphate derived from sulphuric acid, the formate derived from formic acid, the acetate derived from acetic acid, the aconate derived from aconitic acid, the ascorbate derived from ascorbic acid, the benzenesulphonate derived from benzensulphonic acid, the benzoate derived from benzoic acid, the cinnamate derived from cinnamic acid, the citrate derived from citric acid, the embonate derived from embonic acid, the enantate derived from enanthic acid, the fumarate derived from fumaric acid, the glutamate derived from glutamic acid, the glycolate derived from glycolic acid, the lactate derived from lactic acid, the maleate derived from maleic acid, the malonate derived from malonic acid, the mandelate derived from mandelic acid, the methanesulphonate derived from methane sulphonic acid, the naphthalene-2-sulphonate derived from naphtalene-2-sulphonic acid, the phthalate derived from phthalic acid, the salicylate derived from salicylic acid, the sorbate derived from sorbic acid, the stearate derived from stearic acid, the succinate derived from succinic acid, the tartrate derived from tartaric acid, the toluene-p-sulphonate derived from p-toluene sulphonic acid, and the like. Such salts may be formed by procedures well known and described in the art.

Other acids such as oxalic acid, which may not be considered pharmaceutically acceptable, may be useful in the preparation of salts useful as intermediates in obtaining a chemical compound of the invention and its pharmaceutically acceptable acid addition salt.

Additional examples of pharmaceutically acceptable addition salts include, without limitation, the non-toxic inorganic and organic acid addition salts such as the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the, lactate, the maleate, the malonate, the mandelate, the methanesulphonate, the naphthalene-2-sulphonate derived, the phthalate, the salicylate, the sorbate, the stearate, the succinate, the tartrate, the toluene-p-sulphonate, and the like. Such salts may be formed by procedures well known and described in the art.

Metal salts of a chemical compound of the invention include alkali metal salts, such as the sodium salt of a chemical compound of the invention containing a carboxy group.

In the context of this invention the "onium salts" of N-containing compounds are also contemplated as pharmaceutically acceptable salts. Preferred "onium salts" include the alkyl-onium salts, the cycloalkyl-onium salts, and the cycloalkylalkyl-onium salts.

### Labelled Compounds

The compounds of the invention may be used in their labelled or unlabelled form. In the context of this invention the labelled compound has one or more atoms replaced by an atom having an atomic mass or mass number different from the atomic mass or mass number usually found in nature. The labelling will allow easy quantitative detection of said compound.

The labelled compounds of the invention may be useful as diagnostic tools, radio tracers, or monitoring agents in various diagnostic methods, and for *in vivo* receptor imaging.

The labelled isomer of the invention preferably contains at least one radionuclide as a label. Positron emitting radionuclides are all candidates for usage. In the context of this invention the radionuclide is preferably selected from ²H (deuterium), ³H (tritium), ¹³C, ¹⁴C, ¹³¹I, ¹²⁵I, ¹²³I and ¹⁸F.

The physical method for detecting the labelled isomer of the present invention may be selected from Position Emission Tomography (PET), Single Photon Imaging Computed Tomography (SPECT), Magnetic Resonance Spectroscopy (MRS), Magnetic Resonance Imaging (MRI), and Computed Axial X-ray Tomography (CAT), or combinations thereof.

### Methods of Producing Azabicyclo Pyridazinyloxy Derivatives

The enantiomeric azabicyclo pyridazinyloxy derivative of the invention may be prepared by conventional methods for chemical synthesis, e.g. those described in the working examples. The starting materials for the processes described in the present application are known or may readily be prepared by conventional methods from commercially available chemicals.

Also one compound of the invention can be converted to another compound of the invention using conventional methods.

The end products of the reactions described herein may be isolated by conventional techniques, e.g. by extraction, crystallisation, distillation, chromatography, etc.

### Biological Activity

The present invention is devoted to the provision novel ligands and modulators of the nicotinic receptors, which ligands and modulators are useful for the treatment of diseases or disorders related to the cholinergic receptors, and in particular the nicotinic acetylcholine receptor (nAChR). Preferred compounds of the invention show a pronounced nicotinic acetylcholine α7 receptor subtype selectivity.

Due to their pharmacological profile the compounds of the invention may be useful for the treatment of diseases or conditions as diverse as CNS related diseases, PNS related diseases, diseases related to smooth muscle contraction, endocrine disorders, diseases related to neuro-degeneration, diseases related to inflammation, pain, and withdrawal symptoms caused by the termination of abuse of chemical substances.

In a preferred embodiment the compounds of the present invention may be useful for the treatment, prevention or alleviation of a cognitive disorder, learning deficit, memory deficits and dysfunction, Alzheimer's disease, attention deficit, attention deficit hyperactivity disorder (ADHD), Tourette's syndrome, psychosis, depression, Bipolar Disorder, mania, manic depression, schizophrenia, cognitive or attention deficits related to schizophrenia, obsessive compulsive disorders (OCD), panic disorders, eating disorders such as anorexia nervosa, bulimia and obesity, narcolepsy, nociception, AIDS-dementia, senile dementia, autism, Parkinson's disease, Huntington's disease, Amyotrophic Lateral Sclerosis, anxiety, non-OCD anxiety disorders, convulsive disorders, epilepsy, rieurodegenerative disorders, transient anoxia, induced neuro-degeneration, neuropathy, diabetic neuropathy, periferic dyslexia, tardive dyskinesia, hyperkinesia, mild pain, moderate or severe pain, pain of acute, chronic or recurrent character, pain caused by migraine, postoperative pain, phantom limb pain, inflammatory pain, neuropathic pain, chronic headache, central pain, pain related to diabetic neuropathy, to post therapeutic neuralgia, or to peripheral nerve injury, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, jet-lag, arrhythmias, smooth muscle contractions, angina pectoris, premature labour, diarrhoea, asthma, tardive dyskinesia, hyperkinesia, premature ejaculation, erectile difficulty, hypertension, inflammatory disorders, inflammatory skin disorders, acne, rosacea, Crohn's disease, inflammatory bowel disease, ulcerative colitis, diarrhoea, or withdrawal symptoms caused by termination of use of addictive substances, including nicotine containing products such as tobacco, opioids such as heroin, cocaine and morphine, benzodiazepines and benzodiazepine-like drugs, and alcohol.

In a more preferred embodiment the compounds of the invention may be useful for the treatment, prevention or alleviation of pain, mild or moderate or severe pain, pain of acute, chronic or recurrent character, pain caused by migraine, postoperative pain, phantom limb pain, inflammatory pain, neuropathic pain, chronic headache, central pain, pain related to diabetic neuropathy, to post therapeutic neuralgia, or to peripheral nerve injury.

In an even more preferred embodiment the compounds of the invention may be useful for the treatment, prevention or alleviation of diseases, disorders or conditions associated with smooth muscle contractions, convulsive disorders, angina pectoris, premature labour, convulsions, diarrhoea, asthma, epilepsy, tardive dyskinesia, hyperkinesia, premature ejaculation, or erectile difficulty.

In a still more preferred embodiment the compounds of the invention may be useful for the treatment, prevention or alleviation of a neurodegenerative disorder, transient anoxia, or induced neuro-degeneration.

In a yet more preferred embodiment the compounds of the invention may be useful for the treatment, prevention or alleviation of an inflammatory disorder, inflammatory skin disorder, acne, rosacea, Crohn's disease, inflammatory bowel disease, ulcerative colitis, or diarrhoea.

In a further preferred embodiment the compounds of the invention may be useful for the treatment, prevention or alleviation of diabetic neuropathy, schizophrenia, cognitive or attentional deficits related to schizophrenia, or depression.

Finally the compounds of the invention may be useful for the treatment of withdrawal symptoms caused by termination of use of addictive substances. Such addictive substances include nicotine containing products such as tobacco, opioids such as heroin, cocaine and morphine, benzodiazepines, benzodiazepine-like drugs, and alcohol. Withdrawal from addictive substances is in general a traumatic experience characterised by anxiety and frustration, anger, anxiety, difficulties in concentrating, restlessness, decreased heart rate and increased appetite and weight gain.

In this context "treatment" covers treatment, prevention, prophylactics and alleviation of withdrawal symptoms and abstinence as well as treatment resulting in a voluntary diminished intake of the addictive substance.

In another aspect, the compounds of the invention are used as diagnostic agents, e.g. for the identification and localisation of nicotinic receptors in various tissues.

It is at present contemplated that a suitable dosage of the active pharmaceutical ingredient (API) is within the range of from about 0.1 to about 1000 mg API per day, more preferred of from about 10 to about 500 mag API per day, most preferred of from about 30 to about 100 mg API per day, dependent, however, upon the exact mode of administration, the form in which it is administered, the indication considered, the subject and in particular the body weight of the subject involved, and further the preference and experience of the physician or veterinarian in charge.

Preferred compounds of the invention show a biological activity in the sub-micromolar and micromolar range, i.e. of from below 1 to about 100 µM.

### Pharmaceutical Compositions

In another aspect the invention provides novel pharmaceutical compositions comprising a therapeutically effective amount of azabicyclo pyridazinyloxy derivative of the invention.

While a chemical compound of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising the azabicyclo pyridazinyloxy derivative of the invention, or a pharmaceutically acceptable salt or derivative thereof, together with one or more pharmaceutically acceptable carriers therefore, and, optionally, other therapeutic and/or prophylactic ingredients, know and used in the art. The carrier(s) must be "acceptable" in the sense of being compatible with the other ingredients of the formulation and not harmful to the recipient thereof.

The pharmaceutical composition of the invention may be administered by any convenient route, which suits the desired therapy. Preferred routes of administration include oral administration, in particular in tablet, in capsule, in dragé, in powder, or in liquid form, and parenteral administration, in particular cutaneous, subcutaneous, intramuscular, or intravenous injection. The pharmaceutical composition of the invention can be manufactured by any skilled person by use of standard methods and conventional techniques appropriate to the desired formulation. When desired, compositions adapted to give sustained release of the active ingredient may be employed.

Pharmaceutical compositions of the invention may be those suitable for oral, rectal, bronchial, nasal, pulmonal, topical (including buccal and sub-lingual), transdermal, vaginal or parenteral (including cutaneous, subcutaneous, intramuscular, intraperitoneal, intravenous, intraarterial, intracerebral, intraocular infection or infusion) administration, or those in a form suitable for administration by inhalation, or insufflation, including powders and liquid aerosol administration, or by sustained release systems. Suitable examples of sustained release systems include semipermeable matrices of solid hydrophobic polymers containing the compound of the invention, which matrices may be in form of shaped articles, e.g. films or microcapsules.

The chemical compound of the invention, together with a conventional adjuvant, carrier, or diluent, may thus be placed into the form of pharmaceutical compositions and unit dosages thereof. Such forms include solids, and in particular tablets, filled capsules, powder and pellet forms, and liquids, in particular aqueous or non-aqueous solutions, suspensions, emulsions, elixirs, and capsules filled with the same, all for oral use, suppositories for rectal administration, and sterile injectable solutions for parenteral use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

The chemical compound of the present invention can be administered in a wide variety of oral and parenteral dosage forms. It will be obvious to those skilled in the art that the following dosage forms may comprise, as the active component, either a chemical compound of the invention or a pharmaceutically acceptable salt of a chemical compound of the invention.

For preparing pharmaceutical compositions from a chemical compound of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid, which is in a mixture with the finely divided active component.

In tablets, the active component is mixed with the carrier having the necessary binding capacity in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify.

Compositions suitable for vaginal administration may be presented as pessaries, tampons, creams, gels, pastes, foams or sprays containing in addition to the active ingredient such carriers as are known in the art to be appropriate.

Liquid preparations include solutions, suspensions, and emulsions, for example, water or water-propylene glycol solutions. For example, parenteral injection liquid preparations can be formulated as solutions in aqueous polyethylene glycol solution.

The chemical compound according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavours, stabilising and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations, intended for conversion shortly before use to liquid form preparations for oral administration. Such liquid forms include solutions, suspensions, and emulsions. In addition to the active component such preparations may comprise colorants, flavours, stabilisers, buffers, artificial and natural sweeteners, dispersants, thickeners, solubilizing agents, and the like.

For topical administration to the epidermis the chemical compound of the invention may be formulated as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

Compositions suitable for topical administration in the mouth include lozenges comprising the active agent in a flavoured base, usually sucrose and acacia or tragacanth; pastilles comprising the active ingredient in an inert base such as gelatin and glycerine or sucrose and acacia; and mouthwashes comprising the active ingredient in a suitable liquid carrier.

Solutions or suspensions are applied directly to the nasal cavity by conventional means, for example with a dropper, pipette or spray. The compositions may be provided in single or multi-dose form.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronization.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration and continuous infusion are preferred compositions.

Further details on techniques for formulation and administration may be found in the latest edition of Remington's Pharmaceutical Sciences (Maack Publishing Co., Easton, PA).

The actual dosage depends on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 µg/kg i.v. and 1 µg/kg p.o. The upper limit of the dosage range is presently considered to be about 10 mg/kg i.v. and 100 mg/kg p.o. Preferred ranges are from about 0.1 µg/kg to about 10 µg/kg/day i.v., and from about 1 µg/kg to about 100 µg/kg/day p.o.

### Methods of Therapy

The azabicyclo pyridazinyloxy derivatives of the present invention are valuable nicotinic and monoamine receptor modulators, and therefore useful for the treatment of a range of ailments involving cholinergic dysfunction as well as a range of disorders responsive to the action of nAChR modulators.

In another aspect the invention provides a method for the treatment, prevention or alleviation of a disease or a disorder or a condition of a living animal body, including a human, which disease, disorder or condition is responsive to modulation of cholinergic receptors and/or monoamine receptors, and which method comprises administering to such a living animal body, including a human, in need thereof an effective amount of an azabicyclo pyridazinyloxy derivative of the invention.

In a preferred embodiment, the disease, disorder or condition relates to the central nervous system.

In a preferred embodiment, the disease, disorder or condition is anxiety, cognitive disorders, learning deficit, memory deficits and dysfunction, Alzheimer's disease, attention deficit, attention deficit hyperactivity disorder, Parkinson's disease, Huntington's disease, Amyotrophic Lateral Sclerosis, Gilles de la Tourette's syndrome, depression, mania, manic depression, schizophrenia, obsessive compulsive disorders (OCD), panic disorders, eating disorders such as anorexia nervosa, bulimia and obesity, narcolepsy, nociception, AIDS-dementia, senile dementia, periferic neuropathy, autism, dyslexia, tardive dyskinesia, hyperkinesia, epilepsy, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, and jet-lag.

In a another preferred embodiment, the disease, disorder or condition are associated with smooth muscle contractions, including convulsive disorders, angina pectoris, premature labour, convulsions, diarrhoea, asthma, epilepsy, tardive dyskinesia, hyperkinesia, premature ejaculation, and erectile difficulty.

In a third preferred embodiment, the disease, disorder or condition is related to the endocrine system, such as thyrotoxicosis, pheochromocytoma, hypertension and arrhythmias.

In a fourth preferred embodiment, the disease, disorder or condition is a neurodegenerative disorders, including transient anoxia and induced neuro-degeneration.

In a fifth preferred embodiment, the disease, disorder or condition is an inflammatory disorder, including inflammatory skin disorders such as acne and rosacea, Crohn's disease, inflammatory bowel disease, ulcerative colitis, and diarrhoea.

In a sixth preferred embodiment, the disease, disorder or condition is mild, moderate or even severe pain of acute, chronic or recurrent character, as well as pain caused by migraine, postoperative pain, and phantom limb pain.

In a seventh preferred embodiment, the disease, disorder or condition is associated with withdrawal symptoms caused by termination of use of addictive substances, including nicotine containing products such as tobacco, opioids such as heroin, cocaine and morphine, benzodiazepines and benzodiazepine-like drugs, and alcohol.

It is at present contemplated that suitable dosage ranges are 0.1 to 1000 milligrams daily, 10-500 milligrams daily, and especially 30-100 milligrams daily, dependent as usual upon the exact mode of administration, form in which administered, the indication toward which the administration is directed, the subject involved and the body weight of the subject involved, and further the preference and experience of the physician or veterinarian in charge.

A satisfactory result can, in certain instances, be obtained at a dosage as low as 0.005 mg/kg i.v. and 0.01 mg/kg p.o. The upper limit of the dosage range is about 10 mg/kg i.v. and 100 mg/kg p.o. Preferred ranges are from about 0.001 to about 1 mg/kg i.v. and from about 0.1 to about 10 mg/kg p.o.

### EXAMPLES

The invention is further illustrated with reference to the following examples.

### Example 1

### Preparatory Example

All reactions involving air sensitive reagents or intermediates were performed under nitrogen and in anhydrous solvents. Magnesium sulphate was used as drying agent in the workup-procedures and solvents were evaporated under reduced pressure.

### (S)-3-Quinuclidinol free base

Potassium hydroxide (14.7 g, 262 mmol) was added in small portions during 10 minutes to a mixture of 4-nitro-benzoic acid (S)-(1-aza-bicyclo[2.2.2]oct-3-yl) ester fumaric acid salt (33.3 g, 84.9 mmol) and ethanol (99%, 500 ml). The mixture was stirred for 30 minutes at room temperature. The mixture was filtered (4-nitrobenzoic acid was separated). The filtrate was evaporated and combined and stirred with petroleumether (400 ml). The precipitated 4-nitrobenzoic acid was removed by filtration. Once again the filtrate was evaporated and combined and stirred with petroleumether (100 ml). The precipitated 4-nitrobenzoic acid was removed by filtration. Yield 11.2 g (100%).

### 4-Nitro-benzoic acid (S)-(1-aza-bicyclo[2.2.2]oct-3-yl) ester fumaric acid salt (Inversion of stereochemistry from R to S by the Mitsunobu reaction)

A mixture of triphenylphosphine (38.7 g, 147 mmol) and dioxane (100 ml) was cooled to 10°C. Diethylazodicarboxylate (25.7 g, 147 mmol) was added drop-wise at 10°C for 20 minutes. The mixture was stirred for 30 minutes at 10°C. Dioxane (200 ml) was added in order to hinder precipitation.

(R)-3-Quinuclidinol (12.5 g, 98.3 mmol) solved in dioxane (100 ml) was added at room temperature. 4-Nitrobenzoic acid (19.8 g, 118 mmol) was added over a period of 10 minutes at room temperature. The reaction was stirred at room-temperature over night. Fumaric acid salt (12.5 g, 1.1 eq.) and ethanol (150 ml) was added, the volume was reduced to 50% by evaporation. The mixture was heated to a clear solution and was allowed to cool and precipitate for 3 days. The product was isolated by filtration. Yield 34 g (89%). Mp. 177-183°C.

### Method A

### (R)-3-(6-Iodo-pyridazin-3-yloxy)-1-aza-bicyclo[2.2.2]octane fumaric acid salt (Intermediate compound)

(R)-3-Quinuclidinol (7.78 g, 61.2 mmol) was solved in THF (50 ml) and dioxane (150 ml). Potassium *tert*-butoxide (7.61 g, 67.3 mmol) and 3,6-diiodopyridazine (20.3 g, 61.2 mmol), solved in THF (100 ml) was added, followed by stirring for 3 days at room temperature. Water (150 ml) was added. The organic solvent was evaporated. The mixture was extracted with dichloromethane (3 x 75 ml). The organic phase was dried and evaporated. Chromatography on silica gel with dichloromethane, methanol and conc. ammonia (89:10:1) gave the title compound as an oil. Yield 9.43 g (47%). The corresponding salt was obtained by addition of a diethyl ether and methanol mixture (9:1) saturated with fumaric acid. Mp. 277°C.

### (S)-3-(6-Iodo-pyridazin-3-yloxy)-1-aza-bicyclo[2.2.2]octane fumaric acid salt (Intermediate compound)

Was prepared according to Method A from (S)-3-quinuclidinol. Mp. 285°C.

### Method B

### (R)-3-(6-Phenylethynl-pyridazin-3-yloxy)-1-aza-bicyclo[2.2.2]octane fumaric acid salt (Compound B1)

A mixture of (R)-3-(6-iodo-pyridazin-3-yloxy)-1-aza-bicyclo[2.2.2]octane (6.0 g, 18.1 mmol), phenylacetylene (3.98 ml, 36.2 mmol), palladacycle (343 mg, 0.36 mmol), Cul (346 mg, 1.81 mmol), diisopropylethylamine (6.3 ml, 36.2 mmol) and dioxane (60 ml) was stirred for 15 hours at 105°C. The reaction mixture was allowed to reach room-temperature. Aqueous sodium hydroxide (50 ml, 1 M) was added. The organic solvent was evaporated. The mixture was extracted with dichloromethane (3 x 50 ml). Chromatography on silica gel with dichloromethane, methanol and conc. ammonia (89:10:1) gave the title compound as an oil. Yield 0.35 g (19%). The corresponding salt was obtained by addition of a diethyl ether and methanol mixture (9:1) saturated with fumaric acid. Mp. 151.3-157.3°C.

### (R)-3-(6-Thiophen-3-ylethynyl-pyridazin-3-yloxy)-1-aza-biclo[2.2.2]octane (Compound B2)

Is prepared according to Method B from (R)-3-(6-iodo-pyridazin-3-yloxy)-1-aza-bicyclo[2.2.2]octane.

### (R)-3-[6-(4-Methoxy-phenylethynyl)-pyridazin-3-yloxy]-1-aza-bicyclo[2.2.2]octane (Compound B3)

Is prepared according to Method B from (R)-3-(6-iodo-pyridazin-3-yloxy)-1-aza-bicyclo[2.2.2]octane.

### (R)-N-{4-[6-(1-Aza-bicyclo[2.2.2]oct-3-yloxy)-pyridazin-3-ylethynyl]-phenyl}-acetamide (Compound B4)

Is prepared according to Method B from (R)-3-(6-iodo-pyridazin-3-yloxy)-1-aza-bicyclo[2.2.2]octane.

### (R)-4-[6-(1-Aza-bicyclo[2.2.2]oct-3-yloxy)-pryridazin-3-ylethynyl]-phenylamine (Compound B5)

Is prepared according to Method B from (R)-3-(6-iodo-pyridazin-3-yloxy)-1-aza-bicyclo[2.2.2]octane.

### (R)-3-[6-(1-Aza-bicyclo[2.2.2]oct-3-yloxy)-pyridazin-3-ylethynyl]-phenylamine (Compound B6)

Is prepared according to Method B from (R)-3-(6-iodo-pyridazin-3-yloxy)-1-aza-bicyclo[2.2.2]octane.

### (S)-3-(6-Phenylethynyl-pyridazin-3-yloxy-1-aza-bicyclo[2.2.2]octane fumaric acid salt (Compound B7)

Was prepared according to Method B from (S)-3-(6-iodo-pyridazin-3-yloxy)-1-aza-bicyclo[2.2.2]octane. Mp. 154.5-159.4°C.

### (S)-3-(6-Thiophen-3-ylethynyl-pyridazin-3-yloxy)-1-aza-bicyclo[2.2.2]octane (Compound B8)

Is prepared according to Method B from (S)-3-(6-iodo-pyridazin-3-yloxy)-1-aza-bicyclo[2.2.2]octane.

### (S)-3-[6-(4-Methoxy-phenylethynyl)l-pyridazin-3-yloxyl-1-aza-bicyclo[2.2.2]octane (Compound B9)

Is prepared according to Method B from (S)-3-(6-iodo-pyridazin-3-yloxy)-1-aza-bicyclo[2.2.2]octane.

### (S)-N-{4-[6-(1-Aza-bicyclo[2.2.2]oct-3-yloxy)-pyridazin-3-yloxy)-3-ylethynyl]-phenyl}-acetamide (Compound B10)

Is prepared according to Method B from (S)-3-(6-iodo-pyridazin-3-yloxy)-1-aza-bicyclo[2.2.2]octane.

### (S)-4-[6-(1-Aza-bicyclo[2.2.2]oct-3-yloxy)-pyridazin-3-ylethynyl]-phenylamine (Compound B11)

Is prepared according to Method B from (S)-3-(6-iodo-pyridazin-3-yloxy)-1-aza-bicyclo[2.2.2]octane.

### (S)-3-[6-[1-Aza-bicyclo[2.2.2]oct-3-yloxy)-pyridazin-3-ylethynyl]-phenylamine (Compound B12)

Is prepared according to Method B from (S)-3-(6-iodo-pyridazin-3-yloxy)-1-aza-bicyclo[2.2.2]octane.

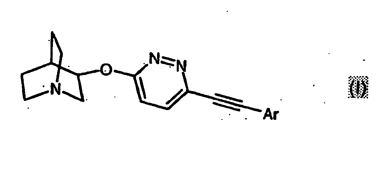

## Claims

1. An enantiopure azabicyclic pyridazinyloxy derivative represented by Formula I 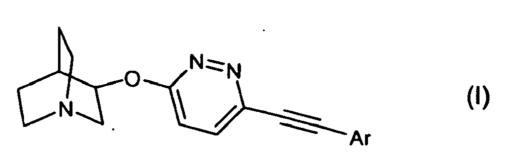 or a pharmaceutically acceptable salt thereof, wherein
Ar represents a phenyl or a thienyl group; which phenyl and thienyl groups are optionally substituted with halo, alkoxy, amino or alkyl-carbonyl-amino.

2. The enantiopure azabicyclic pyridazinyloxy derivative of claim 1, wherein
Ar represents a phenyl group; which phenyl is optionally substituted with halo, alkoxy, amino or alkyl-carbonyl-amino.

3. The enantiopure azabicyclic pyridazinyloxy derivative of claim 1, wherein
Ar represents a thienyl group; which thienyl is optionally substituted with halo, alkoxy, amino or alkyl-carbonyl-amino.

4. The enantiopure azabicyclic pyridazinyloxy derivative of claim 1, which is
(R)-3-(6-Phenylethynyl-pyridazin-3-yloxy)-1-aza-bicyclo[2.2.2]octane;
(R)-3-(6-Thiophen-3-ylethynyl-pyridazin-3-yloxy)-1-aza-bicyclo[2.2.2]octane;
(R)-3-[6-(4-Methoxy-phenylethynyl)-pyridazin-3-yloxy]-1-aza-bicyclo[2.2.2]octane;
(R)-*N*-{4-[6-(1-Aza-bicyclo[2.2.2]oct-3-yloxy)-pyridazin-3-ylethynyl]-phenyl}-acetamide;
(R)-4-[6-(1-Aza-bicyclo[2.2.2]oct-3-yloxy)-pyridazin-3-ylethynyl]-phenyl-amine;
(R)-3-[6-(1-Aza-bicyclo[2.2.2]oct-3-yloxy)-pyridazin-3-ylethynyl]-phenyl-amine;
(S)-3-(6-Phenylethynyl-pyridazin-3-yloxy)-1-aza-bicyclo[2.2.2]octane;
(S)-3-(6-Thiophen-3-ylethynyl-pyridazin-3-yloxy)-1-aza-bicyclo[2.2.2]octane;
(S)-3-[6-(4-Methoxy-phenylethynyl)-pyridazin-3-yloxy]-1-aza-bicyclo[2.2.2]octane;
(S)-N-{4-[6-(1-Aza-bicyclo[2.2.2]oct-3-yloxy)-pyridazin-3-ylethynyl]-phenyl}-acetamide;
(S)-4-[6-(1-Aza-bicyclo[2.2.2]oct-3-yloxy)-pyridazin-3-ylethynyl]-phenyl-amine; or
(S)-3-[6-(1-Aza-bicyclo[2.2.2]oct-3-yloxy)-pyridazin-3-ylethynyl]-phenyl-amine;
or a pharmaceutically acceptable salt thereof.

5. The enantiopure azabicyclic pyridazinyloxy derivative of claim 1, which is
(R)-3-(6-Phenylethynyl-pyridazin-3-yloxy)-1-aza-bicyclo[2.2.2]octane;
or a pharmaceutically acceptable salt thereof.

6. The enantiopure azabicyclic pyridazinyloxy derivative of claim 1, which is
(S)-3-(6-Pheny)ethynyl-pyridazin-3-yloxyl-1-aza-bicyclo[2.2.2]octane;
or a pharmaceutically acceptable salt thereof.

7. A pharmaceutical composition comprising a therapeutically effective amount of an enantiopure azabicyclic pyridazinyloxy derivative of any one of claims 1-6, or a pharmaceutically-acceptable addition salt thereof, together with at least one pharmaceutically-acceptable carrier or diluent.

8. Use of an enantiopure azabicyclic pyridazinyloxy derivative of any one of claims 1-6, or a pharmaceutically-acceptable addition salt thereof, for the manufacture of a pharmaceutical composition/medicament for the treatment, prevention or alleviation of a disease or a disorder or a condition of a mammal, including a human, which disease, disorder or condition is responsive to modulation of cholinergic receptors and/or monoamine receptors.

9. The use according to claim 8, wherein the disease, disorder or condition is a cognitive disorder, learning deficit, memory deficits and dysfunction, Alzheimer's disease, attention deficit, attention deficit hyperactivity disorder (ADHD), Tourette's syndrome, psychosis, depression, Bipolar Disorder, mania, manic depression, schizophrenia, cognitive or attention deficits related to schizophrenia, obsessive compulsive disorders (OCD), panic disorders, eating disorders such as anorexia nervosa, bulimia and obesity, narcolepsy, nociception, AIDS-dementia, senile dementia, autism, Parkinson's disease, Huntington's disease, Amyotrophic Lateral Sclerosis, anxiety, non-OCD anxiety disorders, convulsive disorders, epilepsy, neurodegenerative disorders, transient anoxia, induced neuro-degeneration, neuropathy, diabetic neuropathy, periferic dyslexia, tardive dyskinesia, hyperkinesia, mild pain, moderate or severe pain, pain of acute, chronic or recurrent character, pain caused by migraine, postoperative pain, phantom limb pain, inflammatory pain, neuropathic pain, chronic headache, central pain, pain related to diabetic neuropathy, to post therapeutic neuralgia, or to peripheral nerve injury, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, jet-lag, arrhythmias, smooth muscle contractions, angina pectoris, premature labour, diarrhoea, asthma, tardive dyskinesia, hyperkinesia, premature ejaculation, erectile difficulty, hypertension, inflammatory disorders, inflammatory skin disorders, acne, rosacea, Crohn's disease, inflammatory bowel disease, ulcerative colitis, diarrhoea, or withdrawal symptoms caused by termination of use of addictive substances, including nicotine containing products such as tobacco, opioids such as heroin, cocaine and morphine, benzodiazepines and benzodiazepine-like drugs, and alcohol.

10. An enantiopure azabicyclic pyridazinyloxy derivative of any one of claims 1-6, or a pharmaceutically-acceptable addition salt thereof, for use as a pharmaceutical composition/medicament.

11. An enantiopure azabicyclic pyridazinyloxy derivative of any one of claims 1-6, or a pharmaceutically-acceptable addition salt thereof, for use in the treatment, prevention or alleviation of a disease or a disorder or a condition of a mammal, including a human, which disease, disorder or condition is responsive to modulation of cholinergic receptors and/or monoamine receptors.

## Patentansprüche

1. Enantiomerenreines azabicyclisches Pyridazinyloxy-Derivat, das durch Formel I wiedergegeben ist 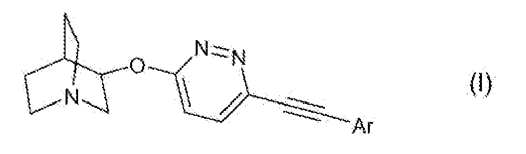 oder ein pharmazeutisch verträgliches Salz davon, worin
Ar eine Phenyl- oder eine Thienylgruppe bedeutet; wobei diese Phenyl- und Thienylgruppen gegebenenfalls mit Halogen, Alkoxy, Amino oder Alkyl-carbonyl-amino substituiert sind.

2. Enantiomerenreines azabicyclisches Pyridazinyloxy-Derivat nach Anspruch 1, worin Ar eine Phenylgruppe bedeutet; wobei dieses Phenyl gegebenenfalls mit Halogen, Alkoxy, Amino oder Alkyl-carbonyl-amino substituiert ist.

3. Enantiomerenreines azabicyclisches Pyridazinyloxy-Derivat nach Anspruch 1, worin Ar eine Thienylgruppe bedeutet; wobei dieses Thienyl gegebenenfalls mit Halogen, Alkoxy, Amino oder Alkyl-carbonyl-amino substituiert ist.

4. Enantiomerenreines azabicyclisches Pyridazinyloxy-Derivat nach Anspruch 1, welches
(R)-3-(6-Phenylethinyl-pyridazin-3-yloxy)-1-aza-bicyclo[2.2.2]octan;
(R)-3-(6-Thiophen-3-ylethinyl-pyridazin-3-yloxy)-1-aza-bicyclo[2.2.2]octan;
(R)-3-[6-(4-Methoxy-phenylethinyl)-pyridazin-3-yloxy]-1-aza-bicyclo[2.2.2]octan;
(R)-*N*-{4-[6-(1-Aza-bicyclo[2.2.2]oct-3-yloxy)-pyridazin-3-ylethinyl]-phenyl}-acetamid;
(R)-4-[6-(1-Aza-bicyclo[2.2.2]oct-3-yloxy)-pyridazin-3-ylethinyl]-phenyl-amin;
(R)-3-[6-(1-Aza-bicyclo[2.2.2]oct-3-yloxy)-pyridazin-3-ylethinyl]-phenyl-amin;
(S)-3-(6-Phenylethinyl-pyridazin-3-yloxy)-1-aza-bicyclo[2.2.2]octan;
(S)-3-(6-Thiophen-3-ylethinyl-pyridazin-3-yloxy)-1-aza-bicyclo[2.2.2]octan;
(S)-3-[6-(4-Methoxy-phenylethinyl)-pyridazin-3-yloxy]-1-aza-bicyclo[2.2.2]octan;
(S)-*N*-{4-[6-(1-Aza-bicyclo[2.2.2]oct-3-yloxy)-pyridazin-3-ylethinyl]-phenyl}-acetamid;
(S)-4-[6-(1-Aza-bicyclo[2.2.2]oct-3-yloxy)-pyridazin-3-ylethinyl]-phenyl-amin; oder
(S)-3-[6-(1-Aza-bicyclo[2.2.2]oct-3-yloxy)-pyridazin-3-ylethinyl]-phenyl-amin ist;
oder ein pharmazeutisch verträgliches Salz davon.

5. Enantiomerenreines azabicyclisches Pyridazinyloxy-Derivat nach Anspruch 1, welches (R)-3-(6-Phenylethinyl-pyridazin-3-yloxy)-1-aza-bicyclo[2.2.2]octan ist;
oder ein pharmazeutisch verträgliches Salz davon.

6. Enantiomerenreines azabicyclisches Pyridazinyloxy-Derivat nach Anspruch 1, welches (S)-3-(6-Phenylethinyl-pyridazin-3-yloxy)-1-aza-bicyclo[2.2.2.]octan ist;
oder ein pharmazeutisch verträgliches Salz davon.

7. Pharmazeutische Zusammensetzung, umfassend eine therapeutisch wirksame Menge von einem enantiomerenreinen azabicyclischen Pyridazinyloxy-Derivat nach einem der Ansprüche 1-6, oder einem pharmazeutisch verträglichen Additionssalz davon, zusammen mit wenigstens einem pharmazeutisch verträglichen Träger oder Verdünnungsmittel.

8. Verwendung von einem enantiomerenreinen azabicyclischen Pyridazinyloxy-Derivat nach einem der Ansprüche 1-6, oder einem pharmazeutisch verträglichen Additionssalz davon, für die Herstellung einer pharmazeutischen Zusammensetzung/eines Medikaments für die Behandlung, Verhütung oder Linderung einer Krankheit oder einer Störung oder eines Zustands bzw. eines Leidens eines Säugers, einschließlich eines Menschen, wobei diese Krankheit, diese Störung oder dieser Zustand bzw. dieses Leiden auf die Modulation von cholinergen Rezeptoren und/oder Monoaminrezeptoren anspricht.

9. Verwendung nach Anspruch 8, wobei es sich bei der Krankheit, der Störung oder dem Zustand bzw. Leiden um eine kognitive Störung, Lerndefizit, Gedächtnisdefizite und - dysfunktion, Alzheimer-Krankheit, Aufmerksamkeitsdefizit, Aufmerksamkeitsdefizit-Hyperaktivitätsstörung (ADHD), Gilles de la Tourette-Syndrom, Psychose, Depression, bipolare Störung, Manie, manische Depression, Schizophrenie, kognitive oder Aufmerksamkeitsdefizite in Verbindung mit Schizophrenie, Zwangsstörungen (OCD), Panikstörungen, Essstörungen wie Anorexia nervosa, Bulimie und Fettleibigkeit, Narkolepsie, Nozizeption, AIDS-Demenz, senile Demenz, Autismus, Parkinson-Krankheit, Chorea Huntington, amyotrophe Lateralsklerose, Angst, Nicht-OCD-Angststörungen, konvulsive Störungen, Epilepsie, neurodegenerative Störungen, vorübergehende Anoxie, induzierte Neurodegeneration, Neuropathie, diabetische Neuropathie, periphere Dyslexie, tardive Dyskinesie, Hyperkinesie, milde Schmerzen, mäßige oder starke Schmerzen, Schmerzen von akutem, chronischem oder wiederkehrendem Charakter, durch Migräne verursachte Schmerzen, postoperative Schmerzen, Phantomschmerzen, entzündliche Schmerzen, neuropathische Schmerzen, chronische Kopfschmerzen, zentrale Schmerzen, Schmerzen in Verbindung mit diabetischer Neuropathie, mit posttherapeutischer Neuralgie oder mit peripherer Nervenverletzung, Bulimie, posttraumatisches Syndrom, Sozialphobie, Schlafstörungen, Pseudodemenz, Ganser-Syndrom, prämenstruelles Syndrom, Syndrom der späten Lutealphase, chronisches Ermüdungssyndrom, Mutismus, Trichotillomanie, Jet-lag, Arrhythmien, Kontraktionen glatter Muskel, Angina pectoris, Frühgeburt, Diarrhö, Asthma, tardive Dyskinesie, Hyperkinesie, vorzeitige Ejakulation, Erektionsschwierigkeiten, Hochdruck, entzündliche Störungen, entzündliche Hautstörungen, Akne, Rosazea, Morbus Crohn, entzündliche Darmerkrankung, Colitis ulcerosa, Diarrhö, oder Entzugssymptome, die durch die Beendigung der Verwendung von süchtig machenden Substanzen, einschließlich nikotinhaltiger Produkte wie Tabak, Opioide wie Heroin, Kokain und Morphin, Benzodiazepine und benzodiazepinartiger Drogen bzw. Arzneimittel, und Alkohol, verursacht werden, handelt.

10. Enantiomerenreines azabicyclisches Pyridazinyloxy-Derivat nach einem der Ansprüche 1-6 oder ein pharmazeutisch verträgliches Additionssalz davon zur Verwendung als eine pharmazeutische Zusammensetzung/ein Medikament.

11. Enantiomerenreines azabicyclisches Pyridazinyloxy-Derivat nach einem der Ansprüche 1 - 6, oder ein pharmazeutisch verträgliches Additionssalz davon, zur Verwendung bei der Behandlung, Verhütung oder Linderung einer Krankheit oder einer Störung oder eines Zustands bzw. eines Leidens eines Säugers, einschließlich eines Menschen, wobei diese Krankheit, diese Störung oder dieser Zustand bzw. dieses Leiden auf die Modulation von cholinergen Rezeptoren und/oder Monoaminrezeptoren anspricht.

## Revendications

1. Dérivé de pyridazinyloxy azabicyclique énantiopur représenté par la formule I 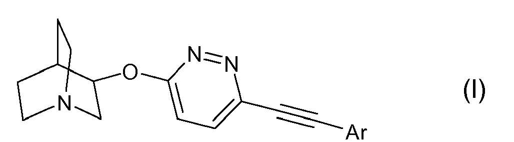 ou un sel pharmaceutiquement acceptable de celui-ci,
dans lequel Ar représente un groupe phényle ou thiényle ; lesquels groupes phényle et thiényle sont facultativement substitués par un halogéno, un alcoxy, un amino ou un alkyl-carbonyl-amino.

2. Dérivé de pyridazinyloxy azabicyclique énantiopur selon la revendication 1, dans lequel Ar représente un groupe phényle ; lequel phényle est facultativement substitué par un halogéno, un alcoxy, un amino ou un alkyl-carbonyl-amino.

3. Dérivé de pyridazinyloxy azabicyclique énantiopur selon la revendication 1, dans lequel Ar représente un groupe thiényle ; lequel thiényle est facultativement substitué par un halogéno, un alcoxy, un amino ou un alkyl-carbonyl-amino.

4. Dérivé de pyridazinyloxy azabicyclique énantiopur selon la revendication 1, qui est
le (R)-3-(6-phényléthynyl-pyridazin-3-yloxy)-1-aza-bicyclo[2.2.2]octane ;
le (R)-3-(6-thiophén-3-yléthynyl-pyridazin-3-yloxy)-1-aza-bicyclo[2.2.2]octane ;
le (R)-3-[6-(4-méthoxy-phényléthynyl)-pyridazin-3-yloxy]-1-aza-bicyclo[2.2.2]octane ;
le (R)-*N*-{4-[6-(1-aza-bicyclo[2.2.2]oct-3-yloxy)-pyridazin-3-yléthynyl]-phényl}-acétamide ;
la (R)-4-[6-(1-aza-bicyclo[2.2.2]oct-3-yloxy)-pyridazin-3-yléthynyl]-phénylamine ;
la (R)-3-[6-(1-aza-bicyclo[2.2.2]oct-3-yloxy)-pyridazin-3-yléthynyl]-phénylamine ;
le (S)-3-(6-phényléthynyl-pyridazin-3-yloxy)-1-aza-bicyclo[2.2.2]octane ;
le (S)-3-(6-thiophén-3-yléthynyl-pyridazin-3-yloxy)-1-aza-bicyclo[2.2.2]octane ;
le (S)-3-[6-(4-méthoxy-phényléthynyl)-pyridazin-3-yloxy]-1-aza-bicyclo[2.2.2]octane ;
le (S)-*N*-{4-[6-(1-aza-bicyclo[2.2.2]oct-3-yloxy)-pyridazin-3-yléthynyl]-phényl}-acétamide ;
la (S)-4-[6-(1-aza-bicydo[2.2.2]oct-3-yloxy)-pyridazin-3-yléthynyl]-phénylamine ; ou
la (S)-3-[6-(1-aza-bicyclo[2.2.2]oct-3-yloxy)-pyridazin-3-yléthynyl]-phénylamine ;
ou un sel pharmaceutiquement acceptable de ceux-ci.

5. Dérivé de pyridazinyloxy azabicyclique énantiopur selon la revendication 1, qui est le (R)-3-(6-phényléthynyl-pyridazin-3-yloxy)-1-aza-bicyclo[2.2.2]octane, ou un sel pharmaceutiquement acceptable de celui-ci.

6. Dérivé de pyridazinyloxy azabicyclique énantiopur selon la revendication 1, qui est le (S)-3-(6-phényléthynyl-pyridazin-3-yloxy)-1-aza-bicyclo[2.2.2]octane, ou un sel pharmaceutiquement acceptable de celui-ci.

7. Composition pharmaceutique comprenant une quantité thérapeutiquement efficace d'un dérivé de pyridazinyloxy azabicyclique énantiopur selon l'une quelconque des revendications 1 à 6, ou d'un sel d'addition pharmaceutiquement acceptable de celui-ci, conjointement avec au moins un véhicule ou diluant pharmaceutiquement acceptable.

8. Utilisation d'un dérivé de pyridazinyloxy azabicyclique énantiopur selon l'une quelconque des revendications 1 à 6, ou d'un sel d'addition pharmaceutiquement acceptable de celui-ci, pour la fabrication d'une composition pharmaceutique/médicament pour le traitement, la prévention ou l'atténuation d'une maladie ou d'un trouble ou d'une affection d'un mammifère, incluant un être humain, laquelle maladie, lequel trouble ou laquelle affection réagit à une modulation des récepteurs cholinergiques et/ou des récepteurs des monoamines.

9. Utilisation selon la revendication 8, dans laquelle la maladie, le trouble ou l'affection est un trouble cognitif, un déficit d'apprentissage, des déficits et un dysfonctionnement de la mémoire, une maladie d'Alzheimer, un déficit de l'attention, un trouble d'hyperactivité avec déficit de l'attention (ADHD), un syndrome de Tourette, une psychose, une dépression, un trouble bipolaire, une manie, une manie-dépression, une schizophrénie, des déficits cognitifs ou de l'attention liés à une schizophrénie, des troubles obsessivo-compulsifs (TOC), des troubles paniques, des troubles de l'alimentation tels qu'une anorexie mentale, une boulimie et une obésité, une narcolepsie, une nociception, une démence liée au SIDA, une démence sénile, un autisme, une maladie de Parkinson, une maladie de Huntington, une sclérose latérale amyotrophique, une anxiété, des troubles d'anxiété non TOC, des troubles convulsifs, une épilepsie, des troubles neurodégénératifs, une anoxie transitoire, une neurodégénérescence induite, une neuropathie, une neuropathie diabétique, une dyslexie périphérique, une dyskinésie tardive, une hyperkinésie, une douleur légère, une douleur modérée ou sévère, une douleur de caractère aigu, chronique ou récurrent, une douleur provoquée par une migraine, une douleur postopératoire, une douleur de membre fantôme, une douleur inflammatoire, une douleur neuropathique, une céphalée chronique, une douleur centrale, une douleur liée à une neuropathie diabétique, à une névralgie post-thérapeutique ou à une blessure de nerf périphérique, une boulimie, un syndrome post-traumatique, une phobie sociale, des troubles du sommeil, une pseudodémence, un syndrome de Ganser, un syndrome prémenstruel, un syndrome de phase lutéale tardive, un syndrome de fatigue chronique, un mutisme, une trichotillomanie, un décalage horaire, des arythmies, des contractions des muscles lisses, une angine de poitrine, un travail prématuré, une diarrhée, un asthme, une dyskinésie tardive, une hyperkinésie, une éjaculation précoce, une difficulté d'érection, une hypertension, des troubles inflammatoires, des troubles cutanés inflammatoires, une acné, une rosacée, une maladie de Crohn, une maladie intestinale inflammatoire, une recto-colite hémorragique, une diarrhée, ou des symptômes de sevrage provoqués par l'arrêt d'utilisation de substances toxicomanogènes, incluant les produits contenant de la nicotine tels que le tabac, les opioïdes tels que l'héroïne, la cocaïne et la morphine, les benzodiazépines et les substances médicamenteuses de type benzodiazépine, et l'alcool.

10. Dérivé de pyridazinyloxy azabicyclique énantiopur selon l'une quelconque des revendications 1 à 6, ou un sel d'addition pharmaceutiquement acceptable de celui-ci, pour une utilisation en tant que composition pharmaceutique/médicament.

11. Dérivé de pyridazinyloxy azabicyclique énantiopur selon l'une quelconque des revendications 1 à 6, ou un sel d'addition pharmaceutiquement acceptable de celui-ci, pour une utilisation dans le traitement, la prévention ou l'atténuation d'une maladie ou d'un trouble ou d'une affection d'un mammifère, incluant un humain, laquelle maladie, lequel trouble ou laquelle affection réagit à une modulation des récepteurs cholinergiques et/ou des récepteurs des monoamines.
